# EUROPEAN PATENT SPECIFICATION

(11) **EP 3 380 175 B1**
(45) Date of publication and mention of the grant of the patent: **24.06.2020**
(21) Application number: 16823333.6
(22) Date of filing: 28.11.2016
(51) Int. Cl.: A61M 11/00, A61M 15/00, A61M 31/00, A61M 35/00, A61M 37/00, B05B 17/00

(54) **DEVICE TO DELIVER A SUBSTANCE**
VORRICHTUNG ZUR VERABREICHUNG EINER SUBSTANZ
DISPOSITIF POUR ADMINISTRER UNE SUBSTANCE

(30) Priority: 26.11.2015 IT UB20155912
(43) Date of publication of application: 03.10.2018
(73) Proprietor: Ferrari Brands S.r.l., 44121 Ferrara (IT)
(72) Inventor: FERRARI, Paolo, 44121 Ferrara (IT)
(74) Representative: Petraz, Davide Luigi
(86) International application number: PCT/IB2016/057154
(87) International publication number: WO 2017/090011

(56) References cited:
- WO-A1-2014/179083
- DE-A1-102012 004 179
- US-A- 3 812 854
- US-A- 5 950 619
- US-A1- 2002 157 677
- US-A1- 2010 083 956
- US-A1- 2014 246 033
- US-B1- 6 196 219
- US-B1- 6 443 146
- US-B1- 6 568 389

## Description

### FIELD OF THE INVENTION

The present invention concerns a device to deliver a substance containing one or more active principles, and the corresponding method.

In particular, the invention is used by way of example in the cosmetic, pharmaceutical, medical or trichological field.

The invention is used by way of example in cutaneous and sub-cutaneous treatments of wounds, lesions and/or various problems, for example acne, inflammations, scars, sores, abrasions, itching, allergies and also in cases of cutaneous dehydration, chapping caused by vasoconstriction, problems with the eyeball etc.

The invention can also be applied for internal treatments of the throat, for vaginal problems or in general for treating internal and external zones of the organism.

### BACKGROUND OF THE INVENTION

To combat the development of wounds, lesions and/or various problems of a cutaneous or sub-cutaneous nature, or also inside the organism, it is known to use a number of substances containing one or more active principles that have high viscosity, also achieving a creamy consistency.

Due to the high viscosity, these known substances can be spread and/or distributed directly on the zone affected by such problems.

Such known substances, if they are diluted with water and/or other substances, can be applied with jets and/or sprays emitted by suitable diffusers, which are associated with the bottles that contain the substances with the active principles in solution therein.

In this latter case, although the substances containing one or more active principles are diluted and then made into drops, the application does not allow to obtain great depths of penetration into the part where the drops are distributed.

In fact, the sizes of the drops produced by known spray diffusers are not small enough to allow to act effectively on the surface or to penetrate effectively inside the surface on which they are applied and consequently to act against the problems that can concern the surface layers of the skin and those below the surface that are to be treated.

Furthermore, using these known methods to apply substances containing one or more active principles, in solution or cream, there are various disadvantages including, for example:
- incorrect dosage of the product with respect to what is actually needed to treat the specific zone of the body;
- non-penetration inside the surface on which the product is applied and/or incorrect distribution on the surface, due to its viscosity and large sizes;
- drying and hence ineffectiveness of the product, located above the first layer deposited, if it does not act quickly;
- inevitable loss of the product which, if applied by spreading it on the surface, largely remains on the palm of the hand.

Another disadvantage of the state of the art is that, in trichological treatments with creams or suchlike, the creams are very obvious on the root of the hair and/or on the scalp of the patient.

In the field of nebulizing substances, nebulization devices are known for cigarettes without emitting smoke, for example described in documents DE-A-10.2012.004.179 and US-A-2014/0246033, which comprise a porous partition through which a substance is forced to pass, for example nicotine. The porous partition is interposed between a first and a second tubular element and is heated by the body heat of the person holding the device in his hand, or by a suitable heating element provided in the porous partition.

A similar device is disclosed also by US-A1-2002/157677, in which the device comprises a plurality of containers for the insulin, made by collapsible walls and covered by a porous membrane provided with a plurality of pores. The collapsible wall can be crushed manually by the user or through a mechanical component, such as a piston that may be driven by a spring, compressed gas or a motor. In this case, a vibration device for energizing the liquid formulation it is provided, for example in the form of piezoelectric ceramic crystal, which generates ultrasonic vibrations at high frequency. Such a device is bulky and complex, especially in the version providing the mechanical crushing of the containers.

Other solutions of nebulization devices are described in documents US-A-3.812.854, US-B1-6.443.146, US-A-5.950.619 and WO-A-2014/179083, which exploit piezoelectric elements to atomize a liquid substance, usually medicinal, which is then aspirated through a pipe and/or a mouthpiece by a user.

However, due to their functioning, all these solutions require the generation of a stream of air, through suction by the user. The stream of air affects the substance to be nebulized and, together with the latter, passes through the porous partition or the piezoelectric elements, to then arrive directly at the user's mouth.

These technical solutions necessarily require a direct suction action by the user to cause the subsequent nebulization of the substance, and cannot be used for distribution of a nebulized flow for example directly onto a surface.

Other prior art devices are disclosed in US.6568.389 B1, US 6.196.219 B1.

Another solution known in the art is disclosed by US-A1-2010/083956, which relates to a discharge head for discharging droplets that is mounted in an inhaler. The discharge head comprises a first orifice plate provided with first discharge ports and a second orifice plate provided with second discharge ports having a diameter smaller than the first discharge ports. The discharge ports of the first and second orifice plates are intended to be passed through in sequence by a pressurized liquid brought to the desired pressure by a piston reciprocating inside a cylinder and driven by a motor through a gearbox. In one version, a piezoelectric body linked to the second orifice plate is provided to confer the droplets passing through the second orifice plate an additional kinetic energy to lower the discharge pressure.

This solution is disadvantageous because it is very complex. In fact, it is clear by this disclosure that many aspects affect the droplets to be discharged, as the liquid pressure, the diameter of the discharge ports, the oscillation frequency of the second orifice plate, and also the distance between the first and the second orifice plates.

There is therefore a need to perfect the state of the art and make available a device to deliver a substance that overcomes at least one of the disadvantages of the state of the art.

Purpose of the present invention is to obtain a portable device, compact and practical, which at the same time allows to apply a substance with an already liquid base, with different parameters of viscosity, containing one or more active principles so that it can act effectively on the surface on which it is applied and/or can also reach deep layers in order to improve its effectiveness.

Another purpose of the present invention is to allow to apply active principles on particular zones, including intimate zones, without contacting said zones with the hand and/or fingers.

The inventive idea allows to use adjustable quantities of the substance with an already liquid base containing one or more active principles, depending on the quantity needed to treat wounds, lesions and/or various problems.

According to the present invention, it is possible on each occasion to rapidly select the liquid based substance/s, also with different viscosities and containing one or more active principles, to be applied on the specific surface, at the same time guaranteeing a more effective treatment.

Another purpose of the present invention is to obtain a device that allows to apply one or more substances with an already liquid base, also with different viscosities, containing trichological active principles so that the application is not obvious.

The Applicant has devised, tested and embodied the present invention to overcome the shortcomings of the state of the art and to obtain these and other purposes and advantages.

### SUMMARY OF THE INVENTION

The present invention is set forth and characterized in the independent claims, while the dependent claims describe other characteristics of the invention or variants to the main inventive idea.

In accordance with the above purposes, a device is provided to deliver one or more substances with a liquid base containing one or more active principles according to claim 1. The device comprises one or more tanks containing the substances, each of which is connected to a nebulizing group configured to nebulize the substance/substances in nebulized particles.

According to some examples described in the present disclosure, the nebulizing group comprises an actuator and a delivery element defining between them a chamber connected fluidically to the at least one tank. The delivery element is provided, in its thickness, with delivery apertures with a maximum diameter of 10 micrometers.

The actuator is chosen from a group comprising a piezoelectric actuator or an ultrasound actuator, and is configured to make the substance contained in the chamber pass through the delivery apertures.

The delivery device also comprises a container in which at least the nebulizing group and the tank are contained. The container is provided with at least a wall with a through aperture in which the nebulizing group is positioned.

According to a variant of the present invention, the device provides more than one tank, each containing a substance with different active principles.

Embodiments of the present invention also concern a method for delivering at least a substance with a liquid base containing one or more active principles at least for cosmetic, pharmaceutical, medical and/or trichological use according to claim 8.

In particular, the method comprises at least the step of:
- supplying the substance in at least one tank, and
- nebulizing the substance by means of a nebulizing group connected to the tank.

In accordance with one aspect of the present invention the method comprises making a nebulizing group available comprising an actuator and a delivery element defining between them a chamber into which the substance is fed from the tank.

In some examples disclosed herein below, the delivery element is provided, in its thickness, with delivery apertures with a maximum diameter of 10 micrometers, and the actuator is chosen from a group comprising a piezoelectric actuator or an ultrasound actuator.

The actuator makes the substance pass through the delivery apertures, and through a through aperture made in a wall of a container containing at least the nebulizing group and the tank.

### BRIEF DESCRIPTION OF THE DRAWINGS

These and other characteristics of the present invention will become apparent from the following description of some embodiments, given as a non-restrictive example with reference to the attached drawings wherein:
- fig. 1 shows a diagram, by way of example, of a device to deliver a substance according to the present invention;
- fig. 2 shows a view from above of a device to deliver a substance according to the present invention;
- fig. 3 shows a section of a device to deliver a substance according to the present invention;
- fig. 4 is a section view of part of a delivery device according to the present invention.

To facilitate comprehension, the same reference numbers have been used, where possible, to identify identical common elements in the drawings. It is understood that elements and characteristics of one embodiment can conveniently be incorporated into other embodiments without further clarifications.

### DETAILED DESCRIPTION OF SOME EMBODIMENTS

With reference to the attached drawings, which show non-restrictive examples of the invention, we will now describe embodiments of a device 10 to deliver a substance 11 in a liquid state, containing one or more active principles.

In particular, the substance 11 can have a viscosity, at room temperature, comprised between 1mPa·s and 100mPa·s, advantageously comprised between 1mPa·s and 50mPa·s, still more advantageously comprised between 1mPa·s and 20mPa·s.

The active principles contained in the substance 11 can be chosen from a group comprising for example hyaluronic acid, aloe extracts, antioxidant substances such as Q10 coenzyme and lipoic acid.

According to the present invention a device 10 comprises at least a tank 12 to contain the substance 11 and a nebulizing group 13 configured to nebulize the substance 11.

The nebulizing group 13 can comprise a chamber 14, an actuator 15 and a delivery element 16.

The chamber 14 is defined by the actuator 15 and the delivery element 16, and is fluidically connected to the at least one tank 12.

According to one possible aspect of the present invention, the tank 12 is a separate and independent element from the nebulizing group 13.

In particular, according to the invention, the actuator 15 and/or the delivery element 16 can be shaped like a disc or thin slab.

According to a possible embodiment of the invention, the actuator 15 and/or the delivery element 16 can have a thickness comprised between 0.1mm and 0.8mm.

According to a possible solution, the actuator 15 can comprise a piezoelectric actuator, an ultrasound actuator or other similar type or which can in any case repeatedly stress the substance 11 so as to nebulize it.

According to possible embodiments of the present invention, the actuator 15 can be the type suitable to generate stresses on the substance 11, for example pressure waves, with a frequency that can reach values equal to 250 kHz.

According to a possible solution of the present invention, the actuator 15 can be facing the delivery element 16 and, when driven, is made to vibrate so as to move alternately into contact with and away from the delivery element 16. The vibrations generated by the actuator 15 oblige the substance 11 to pass through the delivery element 16 as described hereafter.

According to the invention, the delivery element 16 is configured to obtain nebulized particles and to prevent them from re-compacting after they have been nebulized.

According to the present invention, the delivery element 16 is provided in its thickness with delivery apertures 27 through which the substance 11 is made to pass through the action of the actuator 15.

According to possible embodiments of the invention, the delivery apertures 27 can have a maximum diameter of 10 micrometers.

According to a possible solution, the diameter of the delivery apertures 27 can be comprised between 2 micrometers and 10 micrometers, preferably between 4 micrometers and 10 micrometers.

These sizes of the delivery apertures 27 allow to prevent re-compacting of the particles once they have been delivered, and prevent causing a wet effect on the skin.

Furthermore, the reduced size of the delivery apertures 27 prevents the substance 11 present in the chamber 14 from escaping involuntarily through the delivery apertures 27 even if the the actuator 15 is de-activated. Indeed, the reduced sizes take into account the surface tension of the substance 11.

According to the invention, the device 10 comprises a management unit 22 configured to manage the functioning at least of the nebulizing group 13, and in particular to control and regulate the delivery times and modes of the substance 11.

According to a possible embodiment, the management unit 22 can comprise a timer configured to determine the time period for which the substance 11 is delivered.

According to the invention, the operating parameters of the actuator 15, that is, the power of the stress and the duration of the stress, are managed by the management unit 22 so that the already liquid-based substance 11, even after having been nebulized, maintains the chemical-physical characteristics of the active principles.

According to the present invention, the nebulizing process consists in repeatedly stressing the quantity of already liquid-based substance 11, introduced into the chamber 14 by the actuator 15.

In particular, to finely nebulize the already liquid-based substance 11 containing one or more active principles, the actuator 15 generates vibrations and/or mechanical waves such as to overcome the forces of cohesion, or surface tension, which tends to keep the substance 11 compact.

According to possible embodiments, the actuator 15 can be configured to spray the substance 11 in at least two steps:
- a first concentrated step in which the flow has a length comprised between 10 and 15cm;
- a second, more nebulized step in which the substance is dispersed also due to the resistance given by the air, into a less concentrated cloud.

The total length of the jet produced can exceed 50cm before becoming totally dispersed.

The mean diameter of the cloud of product nebulized, measured at a distance of 20cm from the delivery zone, is about 8-10cm.

According to the present invention, the stresses allow to obtain nebulized particles of the substance 11 with diameters in the range of a few tenths of a micrometer.

If the liquid-based substance 11 has high viscosities, the cohesion forces of the liquid that oppose the stresses induced by the actuator 15 are very high.

By way of example, the management unit 22 can comprise at least one of either an integrated circuit, a microcircuit, a microprocessor or other similar device.

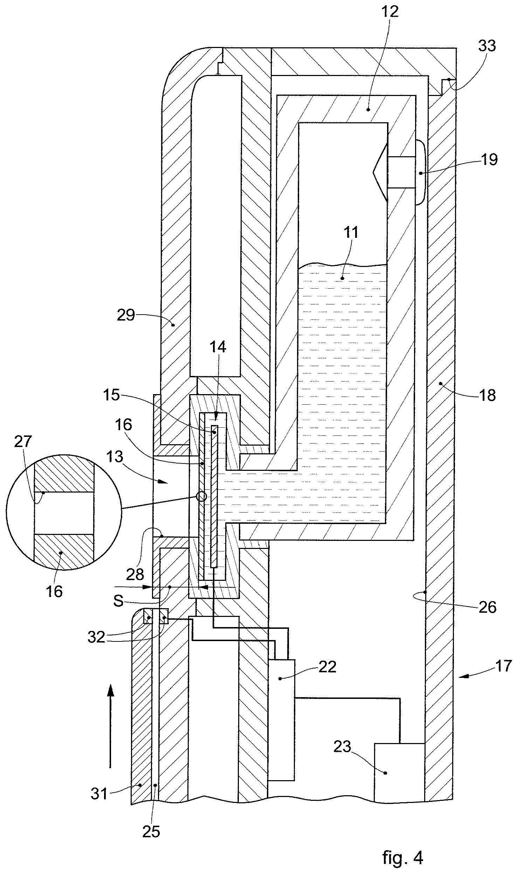

According to a possible embodiment of the present invention, described for example with reference to fig. 4, the nebulizing group 13 can be associated directly with the tank 12, that is, the chamber 14 defined by the actuator 15 and the delivery element 16 is in direct fluidic communication with the tank 12, without any pumping elements or other devices interposed.

The substance 11 is fed to the chamber 14 through gravity.

In particular, it is provided that the tank 12 is provided with an aperture to supply the liquid which is directly connected to the chamber 14.

According to a possible solution, the tank 12 can comprise a V-shaped portion to receive and convey the liquid toward the supply aperture and therefore toward the chamber 14.

According to one embodiment, the device 10 comprises a container 17 defining a compartment 26 in which at least the tank 12 and the nebulizing group 13 are disposed.

The container 17 is provided with at least a through aperture 28 in which the nebulizing group 13 is positioned.

In particular, it can be provided that the through aperture 28 is substantially occluded by the delivery element 16, which faces directly toward the outside, thus allowing a direct delivery of the nebulized substance 11.

The through aperture 28 is made in a wall 29 of the container 17.

The nebulizing group 13 and, in particular, at least the delivery element 16, is directly associated with the wall 29 of the container 17.

Merely by way of example, the through aperture 28 can have a length S comprised between 1mm and 6mm, that is substantially the thickness of the wall 29. These sizes allow to put the delivery element 16 directly toward the outside, preventing the presence of channels that cause the nebulized flow to re-compact and prevent a long-range delivery of the flow.

According to the present invention, the actuator 15 is configured to generate a flow of nebulized substance 11 that exits under pressure also through the through aperture 28.

The through aperture 28 can be defined by an annular element 30, for example as shown in fig. 4.

The container 17 can have specific shapes, depending on the particular application for which the device 10 is intended, by way of example it can have a tapered shape so as to reach the internal zones of the organism, or a flat disc shape to distribute the substance 11 on wide zones, or other shapes. It is obvious that the shape of the container 17 according to the present invention does not restrict the field of protection requested.

The tank 12 can be selectively replaceable and removable from the container 17, for example according to the particular treatment to be performed. In other embodiments, the tank 12 can be the type fixed to the container 17 and is filled on each occasion with the desired substance 11. According to a possible solution of the present invention, the tank 12 can be provided with an aperture, openable/closable with a stopper 19 through which the substance 11 is introduced.

According to the invention, the tank or tanks 12 can be refilled and are advantageously contained inside the container 17 so that they are not exposed to sunlight which in some cases could cause deterioration thereof.

According to a variant, the tank 12 can be disposable, that is, it contains a limited quantity of substance 11 to be nebulized in limited times, depending on the capacity of the substance 11 to maintain its chemical-physical characteristics.

According to a possible solution, the container 17 is provided with an access aperture 33 at least to the tank 12 and to the nebulizing group 13, at least to replace the tank 12 and/or to introduce the substance 11.

The access aperture 33 is selectively openable/closable with a lid 18.

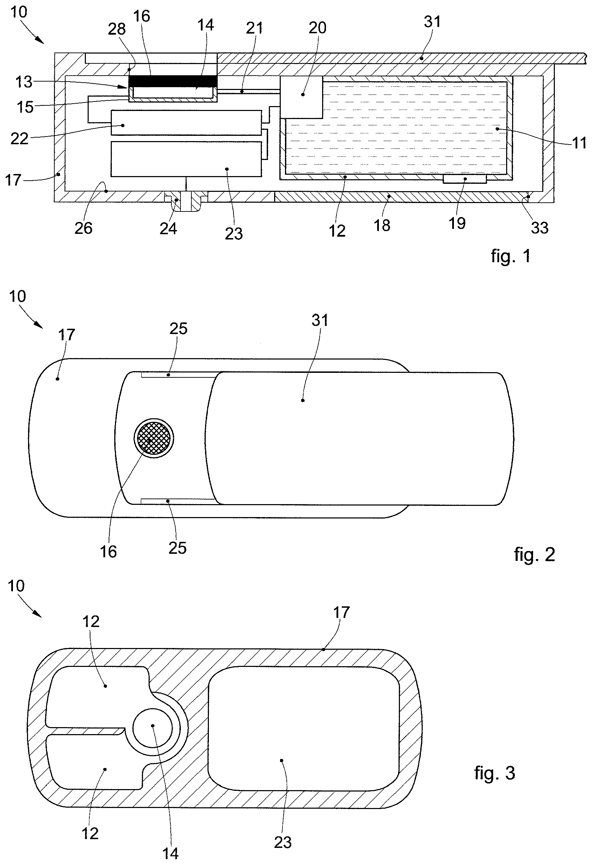

According to one embodiment of the present invention, the device 10 comprises a plurality of tanks 12 (fig. 3) each of which contains a predefined substance 11.

This solution allows to deliver one or more substances at the same time.

According to a possible solution, a respective nebulizing group 13 as described above is associated with each tank 12.

According to a possible solution, it can be provided that the tanks 12 are each provided with respective supply apertures all fluidically connected to the chamber 14 where all the substances 11 are reciprocally mixed for the subsequent nebulized delivery thereof.

In particular, the actuation of the actuator 15 causes the substances to be taken from all the tanks 12 associated with the actuator 15.

The actuator 15 can function as a stopper for the supply apertures of each of the tanks 12 and, when actuated, during vibration, allows the substance 11 to pass toward the chamber 14.

According to a possible embodiment (fig. 1), the nebulizing group 13 can comprise a pumping member 20 connected to the tank 12 and able to remove preset quantities of substance 11 contained in the tank 12 and feed it to the nebulizing group 13.

The pumping member 20 is connected between the tank 12 and the nebulizing group 13 by at least a channel 21.

According to a possible solution of the present invention, the management unit 22 can be connected to the pumping member 20 and to the nebulizing group 13 to coordinate their reciprocal functioning as a function of the predetermined delivery times and modes.

According to a possible solution, to prevent the nebulized substance 11 from re-compacting, the actuator 15 is kept functioning until the whole quantity introduced into the chamber has exited from the delivery apertures 27 of the delivery element 16.

According to a possible solution, and if several tanks 12 are provided, each of them can serve its own pumping member 20. In this variant, the coordinated drive of the pumping members 20 is performed by a single management unit 22.

According to this embodiment, the mixture is obtained inside the chamber 14 or in a mixing chamber, not shown, located upstream of the chamber 14.

Preferably, according to a variant of the present invention, a cleaning liquid is contained in one of the tanks 12 and is used to remove possible blockages of the delivery apertures of the delivery element 16 without damaging the delivery element 16 itself.

Advantageously, the cleaning liquid is a water-based liquid, and even more advantageously the cleaning liquid is water.

According to the present invention, a substance 11 is nebulized by carrying out the following operations:
- activating the actuator 15;
- introducing the substance 11 from the tanks or tanks 12 into the chamber 14;
- forced delivery of the nebulized particles through the delivery apertures of the delivery element 16 by the pressure generated by the actuator 15.

According to the invention, the cooperation of these three factors allows to have a nebulized jet of substance 11 which, also thanks to the delivery speed through the delivery element 16, prevents the nebulized particles from re-compacting to form a single body.

This is made possible by the coordinated combination of the three actions described above, because by nebulizing the substance 11 continuously and delivering the nebulized particles under pressure through the delivery element 16, the nebulized particles do not have the time to re-compact either inside the chamber 14 or after having passed through the delivery apertures 27 of the delivery element 16.

Furthermore, again thanks to the reduced size of the nebulized particles and their consequent rapid absorption, it is possible to prevent stagnation and/or thickening on the surface on which the nebulized substance 11 is applied.

These advantages are more obvious in all those medical and/or cosmetic applications which in the state of the art use creams and/or viscous substances which, to be effective, need to reach the internal layers of the surface on which the substance 11 is applied.

The actuator 15 is fed by an electric energy accumulation device, for example a rechargeable battery 23, such as a lithium ion battery.

Preferably, the rechargeable battery 23 is connected to the management unit 22 to feed the latter.

By way of example, the device 10 comprises a connector or power socket 24 electrically connected to the rechargeable battery 23 and configured to allow to recharge the rechargeable battery 23.

Recharging can be done by connecting the power socket 24 to an external feed, a PC or also to portable feeds provided with solar panels for example.

According to a possible embodiment of the invention, the device 10 is provided with a cover element 31 located to cover the delivery element 16 and, in particular, the through aperture 28, and is attached on the container 17.

The cover element 31 can be mobile on a movement guide 25, for example sliding, made or provided in the container 17.

According to one aspect of the present invention, the movement guide 25 allows the cover element 31 to assume at least two positions: a first closed position that protects the delivery element 16, and a second open position (shown in fig. 2) which allows to have a sufficient free opening for the exit of the nebulized substance or substances 11.

Advantageously, the device allows to activate the delivery of the substance 11 directly, as soon as the cover element 31 is in the second open position. To this purpose at least one of either the cover element 31 or the container 17 can be provided with activation elements 32 connected to the management unit 22 and configured to command the activation of the nebulizing group 13 when the cover element 31 is in its second open position.

The device 10 also allows to nebulize a substance 11 containing one or more active principles for trichological applications.

In this latter case, the particles nebulized by the device 10, as they are distributed and act effectively on the scalp, from the root of the hair, do not form evident layers of substance 11 on the hair.

According to possible solutions, it can be provided that one or more accessories, configured to determine a massaging action on the affected parts, can be associated with the container 17, for example on the wall 29. Merely by way of example, such accessories can be provided with a support body, attachable with snap-in elements to the container 17 and provided with a plurality of protruding portions determining the massaging action on the affected parts.

The support body can be provided with a passage hole to deliver the nebulized substance 11.

It is clear that modifications and/or additions of parts may be made to the device to deliver a substance, and the corresponding method, as described heretofore, without departing from the field and scope of the present invention.

## Claims

1. Device to deliver at least a substance (11) with a liquid base, containing one or more active principles at least for cosmetic, pharmaceutical, medical and/or trichological use, said device comprising:
- at least a tank (12) containing said substance (11),
- a nebulizing group (13) connected to said tank (12) and configured to nebulize said substance (11),
wherein said nebulizing group (13) comprises an actuator (15) and a delivery element (16) defining between them a chamber (14) fluidically connected to the at least one tank (12), said delivery element (16) is provided, in its thickness, with delivery apertures (27) with a maximum diameter of 10 micrometers, said actuator (15) is chosen from a group comprising a piezoelectric actuator or an ultrasound actuator, and is configured to make said substance (11) contained in said chamber (14) pass through said delivery apertures (27), wherein the device comprises a container (17) in which at least said nebulizing group (13) and said tank (12) are contained, said container (17) being provided with at least a wall (29) with a through aperture (28) in which the nebulizing group is positioned (13) and with movement guide (25); said through aperture (28) being occluded by said delivery element (16) which faces directly toward the outside and the device being also **characterized in that** it is provided with a cover element (31) attached on said container (17), placed to cover said delivery element (16) and said through aperture (28), and mobile on said movement guide (25) to assume at least two positions, namely a first closed position that protects said delivery element (16), and a second open position which allows to have a free opening for the exit of said nebulized substance (11); wherein at least one of either said cover element (31) or said container (17) is provided with activation elements (32) connected to a management unit (22) and configured to command the activation of said nebulizing group (13) when said cover element (31) is in said open position.

2. Device as in claim 1, **characterized in that** said through aperture (28) has a length (S) that is comprised between 1mm and 6mm.

3. Device as in any claim hereinbefore, **characterized in that** said chamber (14) is in direct fluidic communication with said tank (12).

4. Device as in any claim hereinbefore, **characterized in that** said tank (12) is provided with an aperture to supply liquid that is directly connected to said chamber (14).

5. Device as in any claim hereinbefore, **characterized in that** said nebulizing group (13) comprises a pumping member (20) connected to said tank (12) and able to remove preset amounts of said substance (11) contained in the tank (12) and to feed it to the nebulizing group (13).

6. Device as in any claim hereinbefore, **characterized in that** it comprises a plurality of said tanks (12) each configured to contain its own type of said substance (11).

7. Device as in any claim hereinbefore, **characterized in that** said at least one tank (12) is selectively replaceable and removable from said device (10).

8. Method for delivering at least a substance (11) with a liquid base containing one or more active principles at least for cosmetic, pharmaceutical, medical and/or trichological use, said method comprising:
- supplying said substance (11) in at least one tank (12),
- nebulizing said substance (11) by means of a nebulizing group (13) connected to said tank (12),
wherein the method comprises making available a nebulizing group (13) comprising an actuator (15) and a delivery element (16) defining between them a chamber (14) in which said substance (11) is fed from said tank (12), said delivery element (16) being provided, in its thickness, with delivery apertures (27) with a maximum diameter of 10 micrometers, and said actuator (15) being chosen from a group comprising a piezoelectric actuator or an ultrasound actuator, wherein said actuator (15) makes said substance (11) pass through said delivery apertures (27), and through a through aperture (28) made in a wall (29) of a container (17) containing at least said nebulizing group (13) and said tank (12) and provided with movement guide (25); said delivery element (16), which faces directly toward the outside, substantially occluding said through aperture (28); said method being also **characterized in that** said movement guide (25) allows a mobile cover element (31), that is attached on said container (17) and placed to cover said delivery element (16) and said through aperture (28), to assume at least two positions, namely a first closed position that protects said delivery element (16), and a second open position which allows to have a free opening for the exit of said nebulized substance (11); wherein the method provides for the activation of said nebulizing group (13) when said cover element (31) is in said open position through activation elements (32) connected to a management unit (22) and configured to command the activation of the nebulizing group (13), said activation elements (32) is provided to at least one of either said cover element (31) or said container (17).

## Patentansprüche

1. Vorrichtung zur Verabreichung mindestens einer Substanz (11) mit einer flüssigen Basis, welche einen oder mehrere aktive Grundbestandteile zumindest für die kosmetische, pharmazeutische, medizinische und/oder trichologische Anwendung enthält, wobei die Vorrichtung umfasst:
- mindestens einen Tank (12), welcher die Substanz (11) enthält,
- eine Vernebelungsgruppe (13), die mit dem Tank (12) verbunden ist und so konfiguriert ist, dass sie die Substanz (11) vernebelt,
wobei die Vernebelungsgruppe (13) einen Aktuator (15) und ein Abgabeelement (16) umfasst, zwischen denen eine Kammer (14) definiert ist, die in Fluidverbindung zu dem mindestens einen Tank (12) steht, wobei das Abgabeelement (16) in seiner Dicke mit Abgabeöffnungen (27) mit einem maximalen Durchmesser von 10 Mikrometern versehen ist, wobei der Aktuator (15) aus einer Gruppe umfassend einen piezoelektrischen Aktuator oder einen Ultraschall-Aktuator ausgewählt ist und so konfiguriert ist, dass er den Durchgang der in der Kammer (14) enthaltenen Substanz (11) durch die Abgabeöffnungen (27) bewirkt,
wobei die Vorrichtung einen Behälter (17) umfasst, in dem zumindest die Vernebelungsgruppe (13) und der Tank (12) enthalten sind, wobei der Behälter (17) mit mindestens einer Wand (29) mit einer Durchgangsöffnung (28), in der die Vernebelungsgruppe (13) angeordnet ist, und mit einer Bewegungsführung (25) versehen ist; wobei die Durchgangsöffnung (28) durch das direkt der Außenseite zugewandte Abgabeelement (16) verschlossen wird, und
wobei die Vorrichtung außerdem **dadurch gekennzeichnet ist, dass** sie mit einem Abdeckungselement (31) versehen ist, das an dem Behälter (17) befestigt ist und so platziert ist, dass es das Abgabeelement (16) und die Durchgangsöffnung (28) abdeckt, und auf der Bewegungsführung (25) beweglich ist, so dass es mindestens zwei Positionen, nämlich eine erste geschlossene Position, welche das Abgabeelement (16) schützt, und eine zweite geöffnete Position, welche eine freie Öffnung zum Austritt der vernebelten Substanz (11) ermöglicht, annehmen kann;
wobei das Abdeckungselement (31) und/oder der Behälter (17) mit Aktivierungselementen (32) versehen ist/sind, die mit einer Steuerungseinheit (22) verbunden sind und so konfiguriert sind, dass sie die Aktivierung der Vernebelungsgruppe (13) befehlen, wenn sich das Abdeckungselement (31) in der geöffneten Position befindet.

2. Vorrichtung gemäß Anspruch 1, **dadurch gekennzeichnet, dass** die Durchgangsöffnung (28) eine Länge (S) zwischen 1 mm und 6 mm aufweist.

3. Vorrichtung gemäß irgendeinem vorhergehenden Anspruch, **dadurch gekennzeichnet, dass** die Kammer (14) in direkter Fluidverbindung mit dem Tank (12) steht.

4. Vorrichtung gemäß irgendeinem vorhergehenden Anspruch, **dadurch gekennzeichnet, dass** der Tank (12) mit einer Öffnung zur Zuführung von Flüssigkeit versehen ist, die direkt mit der Kammer (14) verbunden ist.

5. Vorrichtung gemäß irgendeinem vorhergehenden Anspruch, **dadurch gekennzeichnet, dass** die Vernebelungsgruppe (13) ein Pumpelement (20) umfasst, das mit dem Tank (12) verbunden ist und in der Lage ist, vorgegebene Mengen der in dem Tank (12) enthaltenen Substanz (11) zu entnehmen und sie der Vernebelungsgruppe (13) zuzuführen.

6. Vorrichtung gemäß irgendeinem vorhergehenden Anspruch, **dadurch gekennzeichnet, dass** sie mehrere Tanks (12) umfasst, die jeweils so konfiguriert sind, dass sie ihren eigenen Substanztyp (11) enthalten.

7. Vorrichtung gemäß irgendeinem vorhergehenden Anspruch, **dadurch gekennzeichnet, dass** der mindestens eine Tank (12) selektiv auswechselbar und von der Vorrichtung (10) abnehmbar ist

8. Verfahren zur Verabreichung mindestens einer Substanz (11) mit einer flüssigen Basis, welche einen oder mehrere aktive Grundbestandteile zumindest für die kosmetische, pharmazeutische, medizinische und/oder trichologische Anwendung enthält, wobei das Verfahren umfasst:
- Bereitstellen der Substanz (11) in mindestens einem Tank (12),
- Vernebeln der Substanz (11) mittels einer mit dem Tank (12) verbundenen Vernebelungsgruppe (13),
wobei das Verfahren das Bereitstellen einer Vernebelungsgruppe (13), die einen Aktuator (15) und ein Abgabeelement (16) umfasst, zwischen denen eine Kammer (14) definiert ist, in welche die Substanz (11) von dem Tank (12) zugeführt wird, umfasst, wobei das Abgabeelement (16) in seiner Dicke mit Abgabeöffnungen (27) mit einem maximalen Durchmesser von 10 Mikrometern versehen ist, und wobei der Aktuator (15) aus einer Gruppe umfassend einen piezoelektrischen Aktuator oder einen Ultraschall-Aktuator ausgewählt ist,
wobei der Aktuator (15) den Durchgang der Substanz (11) durch die Abgabeöffnungen (27) und durch eine Durchgangsöffnung (28), die in einer Wand (29) eines Behälters (17) ausgebildet ist, bewirkt, wobei der Behälter (17) zumindest die Vernebelungsgruppe (13) und den Tank (12) enthält und mit einer Bewegungsführung (25) versehen ist; wobei das Abgabeelement (16), welches direkt der Außenseite zugewandt ist, die Durchgangsöffnung (28) im Wesentlichen verschließt,
wobei das Verfahren außerdem **dadurch gekennzeichnet ist, dass** die Bewegungsführung (25) es einem beweglichen Abdeckungselement (31), das an dem Behälter (17) befestigt ist und so angeordnet ist, dass es das Abgabeelement (16) und die Durchgangsöffnung (28) abdeckt, ermöglicht, mindestens zwei Positionen, nämlich eine erste geschlossene Position, welche das Abgabeelement (16) schützt, und eine zweite geöffnete Position, welche eine freie Öffnung zum Austritt der vernebelten Substanz (11) ermöglicht, anzunehmen; wobei das Verfahren die Aktivierung der Vernebelungsgruppe (13) bereitstellt, wenn sich das Abdeckungselement (31) in der geöffneten Position befindet, durch Aktivierungselemente (32), die mit einer Steuerungseinheit (22) verbunden sind und so konfiguriert sind, dass sie die Aktivierung der Vernebelungsgruppe (13) befehlen, wobei die Aktivierungselemente (32) an dem Abdeckungselement (31) und/oder dem Behälter (17) bereitgestellt werden.

## Revendications

1. Dispositif pour délivrer au moins une substance (11) à base liquide, contenant un ou plusieurs principes actifs au moins à usage cosmétique, pharmaceutique, médical et / ou trichologique, ledit dispositif comprenant :
- au moins un réservoir (12) contenant ladite substance (11),
- un groupe de nébulisation (13) connecté audit réservoir (12) et configuré pour nébuliser ladite substance (11),
ledit groupe de nébulisation (13) comprenant un actionneur (15) et un élément de distribution (16) définissant entre eux une chambre (14) reliée fluidiquement audit au moins un réservoir (12), ledit élément de distribution (16) étant pourvu, dans son épaisseur, d'ouvertures de distribution (27) ayant un diamètre maximum de 10 micromètres, ledit actionneur (15) étant choisi dans un groupe comprenant un actionneur piézoélectrique ou un actionneur à ultrasons, et étant configuré pour faire passer ladite substance (11) contenue dans ladite chambre (14) à travers lesdites ouvertures de distribution (27),
le dispositif comprenant un récipient (17) dans lequel au moins ledit groupe de nébulisation (13) et ledit réservoir (12) sont contenus, ledit récipient (17) étant pourvu d'au moins une paroi (29) avec une ouverture traversante (28) dans laquelle le groupe de nébulisation (13) est positionné et avec un guide de mouvement (25) ; ladite ouverture traversante (28) étant obstruée par ledit élément de distribution (16) qui est tourné directement vers l'extérieur, et le dispositif étant également **caractérisé en ce qu'**il est pourvu d'un élément (31) formant couvercle fixé sur ledit récipient (17), placé pour recouvrir ledit élément de distribution (16) et ladite ouverture traversante (28), et mobile sur ledit guide de mouvement (25) pour prendre au moins deux positions, à savoir une première position fermée qui protège ledit élément de distribution (16), et une deuxième position ouverte qui permet d'avoir une ouverture libre pour la sortie de ladite substance nébulisée (11) ; au moins l'un parmi ledit élément (31) formant couvercle et ledit récipient (17) étant pourvu d'éléments d'activation (32) connectés à une unité de gestion (22) et configurés pour commander l'activation dudit groupe de nébulisation (13) lorsque ledit élément (31) formant couvercle est dans ladite position ouverte.

2. Dispositif selon la revendication 1, **caractérisé en ce que** ladite ouverture traversante (28) a une longueur (S) comprise entre 1 mm et 6 mm.

3. Dispositif selon l'une quelconque des revendications précédentes, **caractérisé en ce que** ladite chambre (14) est en communication fluidique directe avec ledit réservoir (12).

4. Dispositif selon l'une quelconque des revendications précédentes, **caractérisé en ce que** ledit réservoir (12) est pourvu d'une ouverture pour fournir du liquide qui est directement connectée à ladite chambre (14).

5. Dispositif selon l'une quelconque des revendications précédentes, **caractérisé en ce que** ledit groupe de nébulisation (13) comprend un élément de pompage (20) relié audit réservoir (12) et apte à soutirer des quantités prédéfinies de ladite substance (11) contenue dans le réservoir (12) et à l'alimenter au groupe de nébulisation (13).

6. Dispositif selon l'une quelconque des revendications précédentes, **caractérisé en ce qu'**il comprend une pluralité desdits réservoirs (12) configurés chacun pour contenir son propre type de ladite substance (11).

7. Dispositif selon l'une quelconque des revendications précédentes, **caractérisé en ce que** ledit au moins un réservoir (12) est sélectivement remplaçable et amovible par rapport audit dispositif (10).

8. Procédé pour délivrer au moins une substance (11) à base liquide contenant un ou plusieurs principes actifs au moins à usage cosmétique, pharmaceutique, médical et / ou trichologique, ledit procédé comprenant :
- le fait d'alimenter ladite substance (11) dans au moins un réservoir (12),
- le fait de nébuliser ladite substance (11) au moyen d'un groupe de nébulisation (13) relié audit réservoir (12),
le procédé comprenant le fait de mettre à disposition un groupe de nébulisation (13) comprenant un actionneur (15) et un élément de distribution (16) définissant entre eux une chambre (14) dans laquelle ladite substance (11) est alimentée à partir dudit réservoir (12), ledit élément de distribution (16) étant pourvu, dans son épaisseur, d'ouvertures de distribution (27) d'un diamètre maximal de 10 micromètres, et ledit actionneur (15) étant choisi dans un groupe comprenant un actionneur piézoélectrique et un actionneur à ultrasons, ledit actionneur (15) permet de faire passer ladite substance (11) à travers lesdites ouvertures de distribution (27), et à travers une ouverture traversante (28) aménagée dans une paroi (29) d'un récipient (17) contenant au moins ledit groupe de nébulisation (13) et ledit réservoir (12) et muni d'un guide de mouvement (25) ; ledit élément de distribution (16), qui est tourné directement vers l'extérieur, obstrue sensiblement ladite ouverture traversante (28) ; ledit procédé étant également **caractérisé en ce que** ledit guide de mouvement (25) permet à un élément mobile (31) formant couvercle, qui est fixé sur ledit récipient (17) et placé pour recouvrir ledit élément de délivrance (16) et ladite ouverture traversante (28), de façon à prendre au moins deux positions, à savoir une première position fermée qui protège ledit élément de distribution (16), et une deuxième position ouverte qui permet d'avoir une ouverture libre pour la sortie de ladite substance nébulisée (11) ; le procédé prévoit l'activation dudit groupe de nébulisation (13) lorsque ledit élément (31) formant couvercle est dans ladite position ouverte au moyen d'éléments d'activation (32) connectés à une unité de gestion (22) et configurés pour commander l'activation du groupe de nébulisation (13), lesdits éléments d'activation (32) étant prévus pour au moins un parmi ledit élément (31) formant couvercle et ledit récipient (17).
